# EUROPEAN PATENT APPLICATION

(11) **EP 2 138 192 A1**
(43) Date of publication of application: **30.12.2009**
(21) Application number: 08720286.7
(22) Date of filing: 27.02.2008
(51) Int. Cl.: A61K 47/42, A61K 9/127, A61K 47/24, B01J 13/04, C07K 14/00

(54) **AGENT FOR ENHANCING THE RESISTANCE OF LIPOSOME AGAINST BIOLOGICAL COMPONENT, AND LIPOSOME MODIFIED WITH THE AGENT**

(30) Priority: 28.02.2007 JP 2007085628
(71) Applicant: National University Corporation Hokkaido University, Sapporo-shi, Hokkaido 060-0808 (JP); President, Kyoto University, Kyoto-shi, Kyoto 606-8501 (JP); Shionogi Co., Ltd., Osaka-shi, Osaka 541-0045 (JP)
(72) Inventor: HARASHIMA, Hideyoshi, Hokkaido 060-0812 (JP); KOGURE, Kentaro, Hokkaido 060-0812 (JP); AKITA, Hidetaka, Hokkaido 060-0812 (JP); IWASA, Akitada, Hokkaido 060-0812 (JP); NAKAMURA, Yoshio, Hokkaido 060-0812 (JP); FUTAKI, Shiroh, Kyoto 611-0011 (JP)
(74) Representative: Prüfer & Partner GbR European Patent Attorneys
(86) International application number: PCT/JP2008/000372
(87) International publication number: WO 2008/105178

(57) **Abstract**

It is intended to provide a positively charged liposome, particularly having a polyarginine peptide on a surface thereof, which is capable of increasing the resistance to a negatively charged biological component such as a protein in the blood and maintaining a high ability to deliver a substance even in the blood. An agent for enhancing the resistance of liposome against biological component comprising a peptide having at least one of the following characteristics (a) and (b) as an active ingredient:
(a) A peptide comprising an amino acid sequence represented by SEQ ID No: 1 or 2; and
(b) A peptide comprising an amino acid sequence represented by SEQ ID No: 1 or 2, in which one or more amino acids are deleted, substituted or added, and having an activity for promoting lipid membrane fusion under acidic condition. An agent for enhancing the resistance of liposome against biological component in this invention, being included in a liposome, is capable of increasing the resistance to a negatively charged biological component such as a protein in the blood and maintaining a high ability to deliver a substance even in the blood.

## Description

### TECHNICAL FIELD

Drug delivery system (DDS) is known to assuredly deliver a pharmaceutical, a nucleic acid, a peptide, a protein, a sugar, etc. to a target cell. As a means thereof, a liposome vector has received much academic attention. Specifically, the liposome vector is advantageous in providing functions, e.g. ensuring the delivery of a substance to a target cell, by introducing functional molecules such as antibody, protein and sugar chain into a surface thereof.

The inventors found that a peptide containing plural consecutive arginine residues (hereinafter called polyarginine peptide) has a function of transferring a substance encapsulated in a liposome having the peptide on a surface thereof into a nucleus (Patent Document 1). Despite this advantage, when a liposome modified with a polyarginine peptide or a liposome mainly containing cationic lipids (both significantly positively charged) is administered in the blood, resulting interaction between the positively charged liposome and a negatively charged biological component in the blood such as a protein reduces the ability of the liposome to deliver a substance. In order to establish a practical positively charged liposome, particularly having a polyarginine peptide on a surface thereof, it is essential to maintain the ability of a liposome to deliver an encapsulated substance even *in vivo.*

Meanwhile, GALA peptide is known as a peptide that can provide a liposome with useful functions (Non-Patent Documents 1 to 4). Specifically, the GALA peptide has a function of promoting lipid membrane fusion in liposomes having the GALA peptide on a surface of a lipid membrane under acidic condition. In addition, the GALA peptide releases a liposome having a GALA peptide on a surface thereof from an endosome to a cytoplasmic fraction after the endosome incorporates the liposome through endocytosis. Unfortunately, the GALA peptide is unable to provide a solution for said problem with the above-described positively charged liposome.
Patent Document 1: International Patent Application Publication No. WO2005-032593
Non-Patent Document 1: T. Kakudo et al., Biochemistry, Vol. 43, pp. 5618-5623, 2004
Non-Patent Document 2: N.K. Subbarao et al., Biochemistry, Vol. 26, pp. 2964-2972, 1987
Non-Patent Document 3: E. Goormaghtigh et al., European J. Biochemistry, Vol. 195, pp. 421-429, 1991
Non-Patent Document 4: R. A. Parente et al., J. Biol. Chem., Vol. 263, pp. 4724-4730, 1988

### DISCLOSURE OF THE INVENTION

### Problem to be solved

It is, therefore, one object of the present invention to provide a positively charged liposome, particularly having a polyarginine peptide on a surface thereof, which is capable of increasing the resistance to a negatively charged biological component such as a protein in the blood and maintaining a high ability to deliver a substance even in the blood.

### Means for solving the problem

The inventors unexpectedly found that a liposome having a GALA peptide on a surface thereof, whose function is to promote lipid membrane fusion of a liposome under acidic condition, has a high ability to deliver an encapsulated substance independent of a negatively charged biological component even in the blood and thus the GALA peptide is usable as an agent for enhancing the resistance of liposome against biological component to complete each of the following inventions.

(1) An agent for enhancing the resistance of liposome against biological component comprising a peptide having at least one of the following characteristics (a) and (b) as an active ingredient:
   (a) A peptide comprising an amino acid sequence represented by SEQ ID No: 1 or 2; and
   (b) A peptide comprising an amino acid sequence represented by SEQ ID No: 1 or 2, in which one or more amino acids are deleted, substituted or added, and having an activity for promoting lipid membrane fusion under acidic condition.

(2) The agent for enhancing the resistance of liposome against biological component according to item (1), in which a peptide in said (a) and/or (b) is modified with a hydrophobic group or a hydrophobic compound.

(3) The agent for enhancing the resistance of liposome against biological component according to item (2), in which said hydrophobic group is a cholesteryl group.

(4) The agent for enhancing the resistance of liposome against biological component according to any one of items (1) to (3), in which a biological component is negatively charged in the blood.

(5) A liposome comprising a peptide having at least one of the following characteristics
   (a) and (b) and a peptide having the following characteristic (c) on a surface thereof:
      (a) A peptide comprising an amino acid sequence represented by SEQ ID No: 1 or 2;
      (b) A peptide comprising an amino acid sequence represented by SEQ ID No: 1 or 2, in which one or more amino acids are deleted, substituted or added, and having an activity for promoting lipid membrane fusion under acidic condition; and
      (c) A peptide containing plural consecutive arginine residues.

(6) The liposome according to item (5), in which a peptide in said (c) contains 4 to 20 consecutive arginine residues.

(7) The liposome according to item (5) or (6), in which a peptide in said (c) comprises only arginine residues.

(8) The liposome according to any one of items (5) to (7), in which a ratio of cationic lipids to total lipids constituting a lipid bilayer is 0 to 40% (molar ratio).

(9) The liposome according to any one of items (5) to (8), in which a peptide in said (a), (b) and/or (c) is modified with a hydrophobic group or a hydrophobic compound, said hydrophobic group or said hydrophobic compound is inserted into a lipid membrane and said peptide is exposed from said lipid membrane.

(10) The liposome according to item (9), in which said hydrophobic group is a stearyl group or a cholesteryl group.

(11) A method for producing a biological component resistance liposome, comprising preparing a liposome comprising a lipid membrane having dioleoylphosphatidylethanolamine and phosphatidic acid.

### BRIEF DESCRIPTION OF THE DRAWINGS

The above and other objects in this invention will be seen by reference to the description taken in connection with the drawings, in which:
Figure 1 shows a gene expression-suppressing effect when siRNA is encapsulated in a liposome in this invention;
Figure 2 shows a gene expression level in a HeLa cell transformed by a liposome in this invention in which a luciferase gene is encapsulated; and
Figure 3 shows a gene expression level in a HeLa cell transformed by a liposome in this invention in which a luciferase gene is encapsulated.

### BEST MODE FOR CARRYING OUT THE INVENTION

An agent for enhancing the resistance of liposome against biological component in this invention comprises (a) a peptide comprising an amino acid sequence represented by SEQ ID No: 1 or 2, or (b) a peptide comprising an amino acid sequence represented by SEQ ID No: 1 or 2, in which one or more amino acids are deleted, substituted or added and having an activity for promoting lipid membrane fusion under acidic condition. The peptide comprising an amino acid sequence represented by SEQ ID No: 1 is known in said Non-Patent Documents 1 to 4, commonly called as GALA peptide. The amino acid sequence represented by SEQ ID No: 2 is a reverse-transcribed sequence of an amino acid sequence of the GALA peptide from N-terminus to C-terminus. A known function of the GALA peptide is an activity for promoting lipid membrane fusion under acidic condition. The peptide comprising an amino acid sequence represented by SEQ ID No: 2 (hereinafter called GALA-R peptide) includes the same function as the GALA peptide. A peptide in (b) is an amino acid variant of the GALA peptide or the GALA-R peptide, having an activity for promoting lipid membrane fusion under acidic condition. The activity can be confirmed by preparing a liposome having a peptide comprising an amino acid sequence in (c) on a surface thereof and observing lipid membrane fusion by putting the liposome under acidic condition.

In this invention, a GALA peptide described in said Non-Patent Documents 1 to 4 and/or the GALA-R peptide can be used as an agent for enhancing the resistance of liposome against biological component. Also, the GALA peptide and/or the GALA-R peptide may be modified with a hydrophobic group or a hydrophobic compound to be used as an agent for enhancing the resistance of liposome against biological component. A hydrophobic group or a hydrophobic compound is not particularly limited if it is inserted into a lipid bilayer of the liposome. Hydrophobic groups may be, e.g. saturated fatty acid groups such as stearyl group or unsaturated fatty acid groups, cholesterol groups or derivatives thereof. Hydrophobic compounds include the above-described phospholipids, glycolipids or sterols, long chain aliphatic alcohols (e.g. phosphatidyl ethanolamine and cholesterol), polyoxypropylene alkyl and glycerine fatty acid ester.

A preferred embodiment in this invention is a GALA peptide and/or a GALA-R peptide modified with a hydrophobic group or a hydrophobic compound. An agent for enhancing the resistance of liposome against biological component can provide a liposome, in which a hydrophobic group or a hydrophobic compound is inserted into a lipid bilayer and a peptide portion of the GALA peptide and/or the GALA-R peptide is exposed from the lipid bilayer. Herein, "peptide is exposed from the lipid bilayer" also means that the peptide is exposed from either an outer surface or an inner surface of the lipid bilayer or both. Preferably, a peptide is exposed from an outer surface of a lipid bilayer in a single membrane liposome and from an outer surface of an outermost lipid bilayer in a multilamellar liposome.

An agent for enhancing the resistance of liposome against biological component in this invention can be used in any liposome, particularly effective in a positively charged liposome. Positively charged liposomes include a cationic lipid and a polyarginine peptide. Also, the agent for enhancing the resistance of liposome against biological component in this invention may be used, e.g. by adding it to a lipid bilayer being formed in the process of liposome preparation and linking it to a surface of the lipid bilayer after formation thereof. A blending amount of the agent for enhancing the resistance of liposome against biological component is not particularly limited, but normally 0.1 to 10% of a total blending amount of a liposome membrane-constituting substance (molar ratio), preferably 0.5 to 5% (molar ratio) and more preferably 0.5 to 2% (molar ratio).

As long as a preferred liposome in the use of an agent for enhancing the resistance of liposome against biological component in this invention is a closed vesicle having a lipid bilayer film structure, the number of lipid bilayers is not particularly limited. The preferred liposome may be multilamellar vesicle (MLV), and single membrane liposomes such as small unilamella vesicle (SUV), large unilamella vesicle (LUV) and giant unilamella vesicle (GUV). Also, the liposome in this invention is not particularly limited in size, but preferably 50 to 800nm in diameter and more preferably 80 to 150nm in diameter.

In a liposome in the use of an agent for enhancing the resistance of liposome against biological component in this invention, the type of a lipid constituting a lipid bilayer is not particularly limited, but specifically one or more of phosphatidyl choline (e.g. dioleoyl phosphatidyl choline, dilauroylphosphatidyl choline, dimyristoylphosphatidyl choline, dipalmitoylphosphatidyl choline and distearoylphosphatidyl choline), phosphatidylglycerol (e.g. dioleoyl phosphatidylglycerol, dilauroylphosphatidylglycerol, dimyristoylphosphatidylglycerol, dipalmitoylphosphatidylglycerol, distearoyl phosphatidylglycerol), phosphatidyl ethanolamine (e.g. dioleoylphosphatidylethanolamine (DOPE), dilauroylphosphatidyl ethanolamine, dimyristoylphosphatidyl ethanolamine, dipalmitoylphosphatidyl ethanolamine, distearoylphosphatidyl ethanolamine), phospholipids such as phosphatidylserine, phosphatidylinositol, phosphatidic acid and cardiolipin or hydrogenated products thereof, glycolipids such as sphingomyelin and ganglioside, and DOPE is a particularly preferable lipid. Phospholipids may be any one of egg yolk, soybean and other naturally occurring lipids derived from animals or plants (e.g. egg yolk lecithin, soybean lecithin), synthetic lipid and semisynthetic lipid.

A lipid bilayer can contain one ore more of animal-derived sterols such as cholesterol, cholesterol succinic acid, lanosterol, dihydrolanosterol, desmosterol and dihydrocholesterol, plant-derived sterols (phytosterol) such as stigmasterol, sitosterol, campesterol and brassicasterol, microorganism-derived sterols such as zymosterol and ergosterol, sugars such as glycerol and sucrose, glycerine fatty acid esters such as triolein and trioctanoin, so as to be physically and chemically stable and set fluidity of a membrane. A content is not particularly limited, but preferably 5 to 40% to total lipids constituting the lipid bilayer (molar ratio), and more preferably 10 to 30% (molar ratio).

A lipid bilayer can contain antioxidant substances such as tocopherol, propyl gallate, ascorbyl palmitate and butylated hydroxytoluene, charged matter that provides a positive charge such as stearylamine and oleylamine, charged matter that provides a negative charge such as dicetyl phosphate, membrane proteins such as membrane extrinsic protein and membrane intrinsic protein, and a content can be adjusted accordingly.

If endocytosis mechanism determines an intracellular transitional path of a liposome, a lipid bilayer must include cationic lipids as a main ingredient, in which an agent for enhancing the resistance of liposome against biological component in this invention is thus advantageous. However, since the intracellular transitional path of a liposome is not actually determined only by endocytosis mechanism, the lipid bilayer doesn't always contain cationic lipids. Consequently, a lipid bilayer of a liposome which can use an agent for enhancing the resistance of liposome against biological component in this invention may constitute either cationic lipids or non-cationic lipids, or both. However, cationic lipids which are prone to cytotoxicity are preferably reduced in volume contained in a lipid bilayer to remove cytotoxicity of a liposome in this invention, and a ratio of cationic lipids to total lipids constituting the lipid bilayer is preferably 0 to 40% (molar ratio) and more preferably 0 to 20% (molar ratio).

Cationic lipids are e.g., dioctadecyldimethylammonium chloride (DODAC), N-(2,3-dioleyloxy) propyl-N,N,N-trimethylammonium (DOTMA), didodecylammonium bromide (DDAB), 1,2-dioleoyloxy-3-trimethylammonio propane, (DOTAP), 3β-N-(N',N',-dimethyl-aminoethane)-carbamol cholesterol (DC-Chol), 1,2-dimyristoyloxypropyl-3-dimethylhydroxyethyl ammonium (DMKIE) and 2,3-dioleyloxy-N-[2(sperminecarboxamido)ethyl]-N,N-dimethyl-1-propanaminum trifluoroacetate, DOSPA.

"Non-cationic lipid" means a neutral lipid or an anionic lipid. Neutral lipids include diacylphosphatidyl choline, diacylphosphatidyl ethanolamine, cholesterol, ceramide, sphingomyelin, cephalin and cerebroside, and anionic lipids include cardiolipin, diacylphosphatidylserine, diacylphosphatidic acid, N-succinylphosphatidyl ethanolamine(N-succinyl PE), phosphatidic acid, phosphatidylinositol, phosphatidylglycerol, phosphatidyl ethanediol and cholesterol succinic acid.

A particularly preferred liposome using an agent for enhancing the resistance of liposome against biological component in this invention can be a liposome having a polyarginine peptide on a surface thereof as disclosed in Patent Document 1. A liposome having a GALA peptide obtained using an agent for enhancing the resistance of liposome against biological component in this invention and a polyarginine peptide partially constitutes this invention.

The number of consecutive arginine residues in a polyarginine peptide is not particularly limited if it is 2 or more, but normally 4 to 20, preferably 6 to 12 and more preferably 7 to 10. The number of amino acid residues overall constituting the above-described peptide is not particularly limited, but normally 4 to 35, preferably 6 to 30 and more preferably 8 to 23. The polyarginine peptide may include any amino acid sequence added to C-terminus and/or N-terminus of plural consecutive arginine residues, but preferably only arginine residues. The most preferable polyarginine peptide is octaarginine peptide composed of only 8 arginine residues.

An amino acid sequence to be added to C-terminus or N-terminus of a polyarginine peptide is preferably a rigid amino acid sequence (e.g. polyproline). Polyproline is a linear and relatively rigid amino acid sequence, as opposed to soft and amorphous polyethylene glycol (PEG).

The ratio of the polyarginine peptide volume contained in a liposome to total lipids constituting a lipid bilayer is normally 0.1 to 30% (molar ratio), preferably 1 to 25% (molar ratio) and more preferably 2 to 20% (molar ratio). If the ratio of the polyarginine peptide volume on a surface of a liposome in this invention to total lipids constituting a lipid bilayer is under 2% (molar ratio), preferably under (molar ratio) and more preferably under 1 % (molar ratio), the liposome can be transferred to a cell mainly via endocytosis. The ratio of the lower limit of the polyarginine peptide volume to total lipids constituting a lipid bilayer is normally 0.1% (molar ratio), preferably 0.5% (molar ratio) and more preferably 0.7% (molar ratio). If the ratio of the polyarginine peptide volume on a surface of a liposome in this invention to total lipids constituting a lipid bilayer is 2% or more (molar ratio), preferably 3% or more (molar ratio) and more preferably 4% or more (molar ratio), the liposome can be transferred to a cell mainly via macropinocytosis. The ratio of the upper limit of the polyarginine peptide volume to total lipids constituting a lipid bilayer is normally 30% (molar ratio), preferably 25% (molar ratio) and more preferably 20% (molar ratio).

As a preferred embodiment in this invention, a liposome can be exemplified, in which said GALA peptide and/or GALA-R peptide and a polyarginine peptide are modified with a hydrophobic group or a hydrophobic compound, the hydrophobic group or the hydrophobic compound is inserted into a lipid bilayer and the above 2 types of the peptides are exposed from the lipid bilayer. Here, "exposed" means that a peptide or a peptide portion is not embedded by the lipid bilayer. Even in cases where said GALA peptide and/or GALA-R peptide and a polyarginine peptide are not modified with a hydrophobic group or a hydrophobic compound, a peptide or a peptide portion is preferably exposed from an outer surface of a lipid bilayer. Also, in a multilamellar liposome, a polyarginine peptide is preferably exposed from an outer surface of each lipid membrane. Each case doesn't rule out that said GALA peptide and/or GALA-R peptide and a polyarginine peptide can be exposed from an inner side of a lipid membrane as well.

A hydrophobic group or a hydrophobic compound is not particularly limited if it can be inserted into a lipid bilayer. A hydrophobic group may be, e.g. saturated fatty acid groups such as stearyl group or unsaturated fatty acid groups, cholesterol group or derivatives thereof, but saturated fatty acid groups whose carbon number is 10 to 20 (e.g. palmitoyl group, oleyl group, stearyl group and arachidoyl group) are particularly preferable. Hydrophobic compound are, e.g. above-exemplified phospholipids, glycolipids or sterols, long chain aliphatic alcohols (e.g. phosphatidyl ethanolamine and cholesterol), polyoxypropylene alkyl and glycerine fatty acid ester.

A liposome using an agent for enhancing the resistance of liposome against biological component in this invention can be prepared by known methods such as hydration, ultrasonic treatment method, ethanol injection technique, ether injection technique, reverse phase evaporation method, surfactant method and freezing and thawing method. For example, in hydration, after lipids constituting a lipid bilayer and GALA peptide and/or GALA-R peptide modified with a hydrophobic group or a hydrophobic compound dissolve in an organic solvent, the organic solvent is evaporated and removed to obtain a lipid membrane. Afterward, the lipid membrane is hydrated, agitated or sonicated to produce a liposome having GALA peptide and/or GALA-R peptide on a surface thereof. In this case, a polyarginine peptide modified with a hydrophobic group or a hydrophobic compound may dissolve in an organic solvent, together with GALA peptide and/or GALA-R peptide modified with a hydrophobic group or a hydrophobic compound.

After lipids constituting a lipid bilayer dissolve in an organic solvent, the organic solvent is evaporated and removed to obtain a lipid membrane. Then, the lipid membrane is hydrated, agitated and sonicated to produce a liposome. Next, GALA peptide and/or GALA-R peptide are added to an external liquid of the liposome to introduce these peptides into a surface of a liposome. The polyarginine peptide may also be added to an external liquid of the liposome together with GALA peptide and/or GALA-R peptide.

In the above method, an organic solvent can be e.g. hydrocarbons such as pentane, hexane, heptane and cyclohexane, halogenated hydrocarbons such as methylene chloride and chloroform, aromatic hydrocarbons such as benzene and toluene, lower alcohols such as methanol and ethanol, esters such as methyl acetate and ethyl acetate, and ketones such as acetone, alone or in combination with each other.

By allowing the organic solvent to pass through a filter with a predetermined pore size, a liposome having a constant particle distribution can be obtained. According to know methods, a multilamellar liposome can be converted into a single membrane liposome and vice verse.

The above-described liposome can encapsulate various bioactive substances such as a pharmaceutical, a nucleic acid, a peptide, a protein, a sugar or complexes thereof, and can be selected according to diagnosis, treatment, etc. If a bioactive substance is soluble, the bioactive substance can be encapsulated in an aqueous phase inside a liposome by adding the bioactive substance to an aqueous solvent used in hydration of a lipid membrane in liposome production. If a bioactive substance is lipid-soluble, the bioactive substance can be encapsulated in a lipid bilayer of a liposome by adding the bioactive substance to an organic solvent used in liposome production.

In addition, a liposome using an agent for enhancing the resistance of liposome against biological component in this invention is advantageous in transferring a complex composed of a nucleic acid and a cationic substance into a cytoplasm and a nucleus. "Cationic substance" is a substance whose molecules include a cationic group which can form a nucleic acid and a complex by electrostatic interaction. The type of cationic substance is not particularly limited if it can form a nucleic acid and a complex, e.g. cationic lipids (e.g. Lipofectamine (Invitrogen)), polymers having a cationic group, polylysine, polyarginine, homopolymer, copolymer or derivatives thereof (e.g. stearyl derivative) of basic amino acid such as a copolymer of lysine and arginine, polycationic polymers such as polyethyleneimine, protamine sulphate, etc. The number of arginine residues constituting a polyarginine is normally 4 to 20, preferably 6 to 12 and more preferably 7 to 10. The number of cationic groups in a cationic substance is not particularly limited, but preferably 2 or more. The cationic group is not particularly limited if it can be positively charged, e.g. monoalkylamino groups such as amino group, methylamino group and ethylamino group, dialkylamino groups such as dimethylamino group and diethylamino group, and imino group, guanidino group, etc. A complex composed of a nucleic acid and a cationic substance is overall positively or negatively charged due to its component ratio, thereby efficiently encapsulating the above complex in the liposome by electrostatic interaction between non-cationic lipids or cationic lipids.

After lipids constituting a lipid bilayer and GALA peptide and/or GALA-R peptide and a polyarginine peptide modified with a hydrophobic group or a hydrophobic compound dissolve in an organic solvent as stated above, an organic solvent is evaporated and removed to obtain a lipid membrane. Since the lipid membrane contains a polyarginine peptide as a cationic substance, electrostatic interaction between a complex composed of said nucleic acid and the cationic substance and lipid membrane can be weak due to its component ratio. In such a case, a lipid membrane containing no polyarginine peptide is preferably used. The lipid membrane containing no polyarginine peptide doesn't dissolve a polyarginine peptide modified with a hydrophobic group or a hydrophobic compound in an organic solvent. After lipids constituting a lipid bilayer dissolve in an organic solvent, the organic solvent is evaporated and removed to obtain the lipid membrane. The polyarginine peptide may be introduced into a liposome surface after a liposome with the above complex encapsulated is formed.

A liposome using an agent for enhancing the resistance of liposome against biological component in this invention can be used e.g. as a form of dispersion. A dispersion medium can be, e.g. buffer solutions such as normal saline solution, phosphate buffer solution, citrate buffer solution and acetate buffer solution. A dispersion may be added as an additive such as sugar, polyhydric alcohol, water-soluble polymer, nonionic surfactant, antioxidant substance, pH adjusting agent and hydration accelerator.

A liposome using an agent for enhancing the resistance of liposome against biological component in this invention can be used with a dispersion dried (e.g. lyophilizated, spray dried, etc.) as well. Dried liposome can be used as dispersion by adding buffer solutions such as normal saline solution, phosphate buffer solution, citrate buffer solution and acetate buffer solution to the dried liposome.

A liposome using an agent for enhancing the resistance of liposome against biological component in this invention can be used both *in vivo* and *in vitro.* When the liposome is used *in vivo,* the route of administration is parenteral administration such as intravenous, intraperitoneal, subcutaneous and intranasal administration. The dosage and number of administration can be adjusted according to the type and volume of a bioactive substance encapsulated in the liposome. This type of liposome can provide intracellular transferability in a wide range of temperature from 0 to 40°C (effectively functioning from 4 to 37°C), thereby determining temperature conditions according to purposes. In particular, if the ratio of the volume of a liposome having a polyarginine peptide produced to total lipids constituting a lipid bilayer is 2% or more (molar ratio), preferably 3% or more (molar ratio) and more preferably 4% or more (molar ratio), the liposome can effectively provide intracellular transferability in a low temperature range (normally 4 to 10°C, preferably 4 to 6°C). The ratio of the upper limit of the polyarginine peptide volume to total lipids constituting a lipid bilayer is normally 30% (molar ratio), preferably 25% (molar ratio) and more preferably 20% (molar ratio).

A liposome using an agent for enhancing the resistance of liposome against biological component in this invention can be used as a vector for intracellular delivery or a vector for intranuclear delivery of an objective substance. Species derived from a cell for delivering the objective substance are not particularly limited, but they may be an animal, a plant, a microorganism, etc., but preferably an animal and more preferably a mammal. Mammals include human, monkey (ape), cattle, sheep, goat, horse, swine, rabbit, dog, cat, rat, mouse and guinea pig. The type of cell for delivering an objective substance is not particularly limited, e.g. somatic cell, generative cell, stem cell or cultured cell thereof.

### EXAMPLE

### <Example 1>

An amide body of a GALA peptide comprising an amino acid sequence represented by SEQ ID No: 1 was chemically synthesized and refined using a peptide synthesizer according to a method described in Non-Patent Document 1 and a C-terminus amide body was subjected to cholesteryl reaction. 64.2µl of 10mM DOPE, 18.35µl of 10mM PA (DOPE:PA=7:2) and 1mM of cholesteryl GALA peptide were dispensed into glass test tubes so that molar concentrations were 0%, 1mol% (8.25µL) and 2mol% (16.5µL), respectively. After 200µl of chloroform was added thereto and dissolved, nitrogen gas was blown to evaporate and dry the product to form a lipid membrane. 1.5mL of DEPC-treated water was added to the lipid membrane to hydrate the lipid membrane for 10 minutes. Next, the product was sonicated for approx. 1 minute using a water tank type ultrasonic wave generating apparatus to prepare a multilamellar liposome. Then, the product was sonciated for 10 minutes using a probe-type ultrasonic wave generating apparatus to prepare a small single membrane liposome (SUV). Afterward, centrifugal separation (15000rpm, 20°C, 5min) was repeated 3 times to obtain an SUV suspension (lipid concentration: 0.55mM) to remove metal piece (titanium) of a probe. A solution obtained by adding 72µL of DEPC-treated water to 18µL of 0.5mg/mL siRNA (Greiner bio-one) and a solution obtained by adding 171µL of DEPC-treated water to 9µL of 2mg/mL stearyl octaarginine were mixed to prepare a condensed siRNA suspension. The SUV suspension and condensed siRNA suspension (both volumes unprescribed) were mixed with a ratio of 2:1 (volume ratio). Anti-luciferase siRNA was encapsulated by vortexing to obtain 3 types of liposomes (GALAR8 liposome) having an octaarginine and a GALA peptide having different GALA peptide contents.

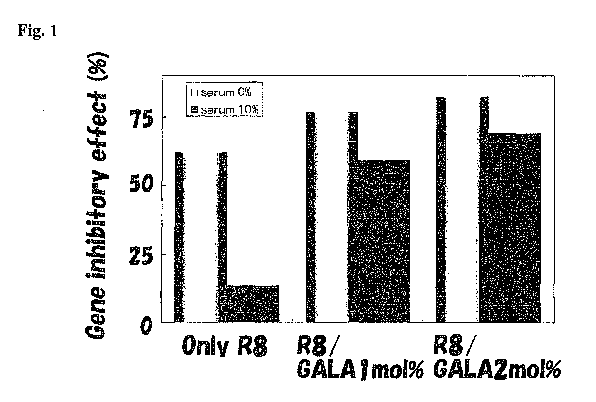

HeLa cells transformed by luciferase gene (4×10⁴cells/well) were seeded into a 24-well plate, to which 0.25mL of 4 types of GALAR8 liposomes and cow embryo-derived serum were added so that the final concentration reached 10%, and cultured under 5%CO₂ condition at 37°C for 3 hours. 1mL of DMEM medium containing serum 10% was further added to each well and cultured under 5%CO₂ condition at 37°C for 21 hours culture to collect cells and determine the volumes of luciferase activity and protein. An R8 liposome containing no GALA peptide (0mol%) in the presence of 10% serum showed no significant effect of suppressing luciferase activity, but GALAR8 liposomes having GALA peptides (1mol%, 2mol%) showed a significant effect of suppressing luciferase activity. In particular, a GALAR8 liposome having 2mol% GALA peptide demonstrated luciferase activity reduction by 70% or more (Figure 1).

<Example 2> 55µL of 5mM DOPE and 275µL of 1mM CL (DOPE:CL=5:5) were dispensed into glass test tubes. 125µL of chloroform was added thereto and mixed, and nitrogen gas was blown to evaporate and dry the product to form a lipid membrane. 1mL of 10mM HEPES buffer solution was dropped into a lipid membrane and allowed to stand to be hydrated at room temperature for 10 minutes. The hydrated product was sonicated for 10 minutes using a probe-type ultrasonic wave generating apparatus to prepare a single membrane liposome (SUV) (SUV-A). 42.35µL of 5mM DOPE, 6.05µL of 10mM PA (DOPE:PA=7:2) and 1mM cholesteryl GALA peptide were dispensed into glass test tubes, so that molar concentrations were 0mol%, 1mol%, 2mol% and 4mol%. After 125µL of chloroform was added to each product and dissolved, nitrogen gas was blown to evaporate and dry the product to form a lipid membrane. 495µL of 10mM HEPES buffer solution was dropped into the lipid membrane and allowed to stand to be hydrated at room temperature for 10 minutes. The hydrated product was sonicated for 10 minutes using a probe-type ultrasonic wave generating apparatus to prepare a single membrane liposome (SUV-B).

An HEPES solution of pEGFPLuc (BD Biosciences Clontech) was dropped into an HEPES solution containing a protamine to prepare a condensed DNA suspension (pEGFPLuc:protamine=2.2:1).
SUV-A (DOPE/CL) and condensed DNA suspension were mixed at a rate of 2:1 to coat the condensed DNA with a lipid double membrane by vortexing (DNA-encapsulating liposome 1). A stearyl octaarginine peptide equivalent to 20mol% of total lipids was added to the DNA-encapsulating liposome 1 and incubated at room temperature for 30 minutes to obtain a stearyl octaarginine-modified DNA-encapsulating liposome 1. Subsequently, the stearyl octaarginine-modified DNA-encapsulating liposome 1 and SUV-B (DOPE/PA/GALA) were mixed at a rate of 1:2 to coat the stearyl octaarginine-modified DNA-encapsulating liposome 1 with a lipid double membrane by vortexing (DNA-encapsulating liposome 2).

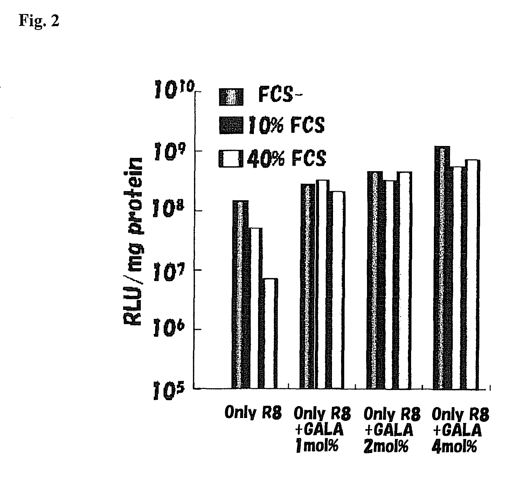

A stearyl octaarginine peptide equivalent to 10% of total lipids was added to the DNA-encapsulating liposome 2 and incubated at room temperature for 30 minutes to obtain a stearyl octaarginine-modified DNA-encapsulating liposome 2. The day before the experiment, HeLa cells (4×10⁴cells/well/DMEM+10% FCS) were seeded into a 24-well plate. After washing the cells with PBS, 410 µL of DMEM, DMEM+10% FCS, DMEM+40% FCS were each added to a well, 4 types of stearyl octaarginine-modified DNA-encapsulating liposome 2 were added thereto, and cultured under 5%CO₂ condition at 37°C for 3 hours. 3 hours later, the cells were washed with PBS, and the medium was exchanged for 500µl of DMEM+10% FCS medium and cultured under 5%CO₂ condition at 37°C for 21 hours. After culturing, the cells were collected, 75µL of 1×reporter lysis buffer was added thereto, and allowed to stand to be frozen at -80°C for 30 minutes or more. After unfreezing the cells, they were collected with a cell scraper, and 20µL of supernatant of cell suspension was mixed with 50µL of luciferase substrate to measure luciferase activity using a luminometer. The luciferase activity was corrected as an activity per protein content by measuring a protein content of the supernatant. While the stearyl octaarginine-modified DNA-encapsulating liposome 2 containing no GALA peptide shows a decline in gene expression as serum concentration increases (reduced to 1/500 gene expression in the presence of 40% serum concentration), the stearyl octaarginine-modified DNA-encapsulating liposome 2 that modified GALA peptide showed gene expression activity, even in the presence of serum, which was equivalent to that with no serum (Figure 2).

<Example 3> 17.1µL of 5mM DOPE, 2.44µL of 10mM PA (DOPE:PA=7:2) and 1mM cholesteryl GALA peptide were dispensed into glass test tubes so that molar concentrations were 0mol%, lmol%, 2mol% and 4mol%. After 125µL of chloroform was added thereto and dissolved, nitrogen gas was blown to evaporate and dry the product to form a lipid membrane.

An HEPES solution of pEGFPLuc (BD Biosciences Clontech) was dropped into an HEPES solution containing a protamine to prepare a condensed DNA suspension (pEGFPLuc:protamine=2.2:1).
200µL of said condensed DNA suspension was added to the lipid membrane and allowed to stand to be hydrated at room temperature for 10 minutes. The hydrated product was sonicated (for several seconds) using an ultrasonic tank to encapsulate a luciferase gene and obtain 4 types of DNA-encapsulating liposomes having different GALA peptide contents.
A stearyl octaarginine peptide equivalent to 10mol% of total lipids was added to the DNA-encapsulating liposome and incubated at room temperature for 30 minutes to prepare a liposome having octaarginine peptide and GALA peptide (0 to 4mol%) (GALAR8 DNA-encapsulating liposome).

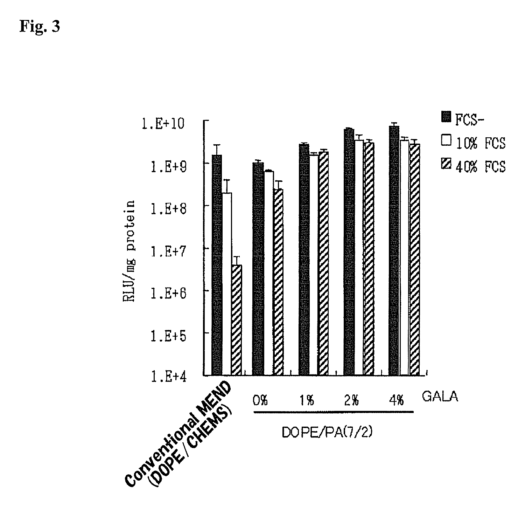

The day before the experiment, HeLa cells (4×10⁴cells/well/DMEM+10% FCS) were seeded into a 24-well plate. After the cells were washed with PBS, 240µL of DMEM, DMEM+10% FCS, DMEM+40% FCS were each added to a well. 4 types of GALAR8 DNA-encapsulating liposomes were added thereto, and cultured under 5% CO₂ condition at 37°C 3 hours. 3 hours later, the cells were washed with PBS, the medium was exchanged for 500µl of DMEM+10% FCS medium and cultured under 5% CO₂ condition at 37°C for 21 hours. After culturing, the cells were collected, 75µL of 1×reporter lysis buffer was added thereto to allow to stand to be frozen at -80°C for 30 minutes or more. After unfreezing it, the cells were collected using a cell scraper, 20µL of supernatant of cell suspension and 50µL of luciferase substrate were mixed to measure the luciferase activity using a luminometer. The luciferase activity was corrected as activity per protein content by measuring protein content of the supernatant.
While the DNA-encapsulating liposome containing no GALA peptide showed approx. 1/4 luciferase gene expression reduction even in the presence of 40% serum concentration by serum components and increase in resistance to serum by DOPE and PA, the DNA-encapsulating liposome that modified GALA peptide showed that serum resistance was completely overcome even in the presence of serum and gene expression was more active (Figure 3).

### INDUSTRIAL APPLICABILITY

An agent for enhancing the resistance of liposome against biological component in this invention, being included in a liposome, is capable of increasing the resistance to a negatively charged biological component such as a protein in the blood and maintaining a high ability to deliver a substance even in the blood. In particular, by adding an agent for enhancing the resistance of liposome against biological component in this invention to a liposome having a polyarginine peptide, functions of the polyarginine peptide can be maintained even with the liposome in the blood.

Sequence listing

## Claims

1. An agent for enhancing the resistance of liposome against biological component comprising a peptide having at least one of the following characteristics (a) and (b) as an active ingredient:
(a) A peptide comprising an amino acid sequence represented by SEQ ID No: 1 or 2; and
(b) A peptide comprising an amino acid sequence represented by SEQ ID No: 1 or 2, in which one or more amino acids are deleted, substituted or added, and having an activity for promoting lipid membrane fusion under acidic condition.

2. The agent for enhancing the resistance of liposome against biological component as set forth in Claim 1, in which a peptide in said (a) and/or (b) is modified with a hydrophobic group or a hydrophobic compound.

3. The agent for enhancing the resistance of liposome against biological component as set forth in Claim 2, in which said hydrophobic group is a cholesteryl group.

4. The agent for enhancing the resistance of liposome against biological component as set forth in any one of Claims 1 to 3, in which a biological component is negatively charged in the blood.

5. A liposome comprising a peptide having at least one of the following characteristics
(a) and (b) and a peptide having the following characteristic (c) on a surface thereof:
(a) A peptide comprising an amino acid sequence represented by SEQ ID No: 1 or 2;
(b) A peptide comprising an amino acid sequence represented by SEQ ID No: 1 or 2, in which one or more amino acids are deleted, substituted or added, and having an activity for promoting lipid membrane fusion under acidic condition; and
(c) A peptide containing plural consecutive arginine residues.

6. The liposome as set forth in Claim 5, in which a peptide in said (c) contains 4 to 20 consecutive arginine residues.

7. The liposome as set forth in Claim 5 or 6, in which a peptide in said (c) comprises only arginine residues.

8. The liposome as set forth in any one of Claims 5 to 7, in which the ratio of cationic lipids to total lipids constituting a lipid bilayer is 0 to 40% (molar ratio).

9. The liposome as set forth in any one of Claims 5 to 8, in which a peptide in said (a), (b) and/or (c) is modified with a hydrophobic group or a hydrophobic compound, said hydrophobic group or said hydrophobic compound is inserted into a lipid membrane and said peptide is exposed from said lipid membrane.

10. The liposome as set forth in Claim 9, in which said hydrophobic group is a stearyl group or a cholesteryl group.

11. A method for producing a biological component resistance liposome, comprising preparing a liposome comprising a lipid membrane having dioleoylphosphatidylethanolamine and phosphatidic acid.
